# FASCICULE DE BREVET EUROPEEN

(11) **EP 1 559 083 B1**
(45) Date de publication et mention de la délivrance du brevet: **21.02.2007**
(21) Numéro de dépôt: 03778454.3
(22) Date de dépôt: 24.10.2003
(51) Int. Cl.: G09F 9/35, B65F 3/14

(54) **Dispositif d'affichage électronique et dispositif de distribution de produit fluide comportant un tel dispositif d'affichage**
Elektronische Anzeigevorrichtung und Flüssigkeitsverteilervorrichtung mit einer solchen Anzeigevorrichtung
Electronic display device and fluid dispensing device comprising the same

(30) Priorité: 28.10.2002 FR 0213470
(43) Date de publication de la demande: 03.08.2005
(73) Titulaire: Valois SAS, 27110 Le Neubourg (FR)
(72) Inventeur: BRUNA, Pascal, F-76300 Sotteville les Rouen (FR)
(74) Mandataire: CAPRI
(86) Numéro de dépôt international: PCT/FR2003/003159
(87) Numéro de publication internationale: WO 2004/040536

(56) Documents cités:
- EP-A- 1 098 235
- GB-A- 2 191 326
- US-B1- 6 188 742

## Description

La présente invention concerne un dispositif d'affichage électronique, ainsi qu'un dispositif de distribution de produit fluide comportant un tel dispositif d'affichage.

Les dispositifs d'affichage électroniques sont largement utilisés dans un grand nombre de domaines techniques. Un domaine d'utilisation particulier est formé par des indicateurs de doses utilisés avec des dispositifs de distribution de produit fluide, notamment dans le domaine pharmaceutique. Ces indicateurs de doses permettent notamment d'informer l'utilisateur du nombre de doses distribuées ou restant à distribuer. Dans ce type d'utilisation, un signal électrique est généralement généré lors de la délivrance de la dose, c'est-à-dire lors de l'actionnement du distributeur, ce signal électrique étant ensuite traité électroniquement et transféré à un afficheur électronique pour modifier l'affichage, en l'occurrence compter ou décompter une dose. Les afficheurs sont généralement constitués d'afficheur à cristaux liquides (LCD : Liquid Cristal Display). Ces indicateurs, et plus généralement les dispositifs d'affichage électronique, utilisent obligatoirement une source d'énergie électrique pour fonctionner, qui est généralement une pile, un accumulateur, ou éventuellement une liaison au secteur. Ce type de source d'énergie est relativement coûteux à réaliser et à installer, ce qui augmente d'autant le coût de fabrication et d'utilisation du distributeur de médicament. Une électronique de contrôle également coûteuse est notamment nécessaire pour commander et gérer la source d'énergie.

La présente invention a pour but de fournir un dispositif d'affichage électronique qui ne reproduit pas les inconvénients susmentionnés.

La présente invention a également pour but de fournir un distributeur de produit fluide comportant un indicateur de doses qui ne reproduit pas les inconvénients susmentionnés.

Plus particulièrement, la présente invention a pour but de fournir un dispositif d'affichage électronique qui soit simple et peu coûteux à fabriquer et à assembler.

La présente invention a également pour but de fournir un tel dispositif d'affichage électronique qui soit peu encombrant, et facilement adaptable à tous dispositifs de distribution de produit fluide existants, sans en modifier sensiblement les dimensions.

La présente invention a également pour but de fournir un tel dispositif d'affichage qui fonctionne de manière fiable, quelle que soit la durée d'utilisation ou de stockage du dispositif, en étant indépendant d'une source d'énergie d'alimentation pour faire fonctionner ledit dispositif.

La présente invention a donc pour objet un dispositif d'affichage électronique selon la revendication 1.

Avantageusement, ledit afficheur est du type à cristaux liquides (LCD).

Avantageusement, ledit afficheur comporte des cristaux nématiques bistables.

Avantageusement, ledit dispositif d'affichage fait partie d'un indicateur ou compteur de doses d'un dispositif de distribution de produit fluide.

La présente invention a également pour objet un dispositif de distribution de produit fluide comportant un corps, un réservoir de produit fluide, un organe de distribution, tel qu'une pompe ou une valve, et un indicateur de doses pour compter le nombre de doses distribuées ou restant à distribuer du réservoir, ledit compteur de doses comportant un dispositif d'affichage tel que décrit ci-dessus.

Avantageusement, l'impulsion électrique nécessaire pour modifier l'affichage est créée par un percuteur déplacé contre un contacteur lors de l'actionnement du dispositif de distribution.

Avantageusement, ledit contacteur est fixe par rapport au corps et ledit percuteur coopère avec un ressort.

D'autres caractéristiques et avantages de la présente invention apparaîtront plus clairement au cours de la description détaillée suivante d'un mode de réalisation particulier de la présente invention, fait en référence aux dessins joints, donnés à titre d'exemple non limitatifs, et sur lesquels
La figure 1 est une vue schématique de côté en section transversale d'un distributeur de produit fluide selon la présente invention ;
La figure 2 est un schéma de principe du dispositif d'affichage selon un mode de réalisation de la présente invention.

Un des objectifs principaux de la présente invention est de réaliser un afficheur qui consomme un minimum d'énergie, et qui est indépendant d'une source d'énergie d'alimentation, de sorte qu'il n'y a aucun risque que cette source soit épuisée, ce qui peut arriver avec des piles ou des accumulateurs, notamment lorsque les temps de stockage ou d'utilisation sont très longs. De même, toute électronique de contrôle et de gestion de la source d'énergie est supprimée.

Le dispositif électronique d'affichage de la présente invention utilise donc un afficheur 21 du type permanent, c'est-à-dire qu'aucune énergie n'est nécessaire pour maintenir l'affichage inchangé, et seule une très faible énergie est nécessaire pour modifier cet affichage. Ce type d'afficheur peut être du type à cristaux liquides (LCD), et plus particulièrement, l'afficheur 21 comporte de préférence des cristaux nématiques bistables.

Pour créer l'énergie nécessaire pour modifier l'affichage de l'afficheur 21, la présente invention prévoit d'utiliser l'interaction entre deux éléments qui se déplacent l'un par rapport l'autre. Une telle interaction peut par exemple être constituée d'un frottement ou d'une percussion entre ces deux éléments. De préférence, un convertisseur électromécanique est utilisé pour transformer cette interaction en une impulsion électrique. Comme convertisseur électromécanique, on peut citer un générateur ou actionneur piézoélectrique, une bobine électromagnétique ou tout autre dispositif de conversion électromécanique connu par l'homme du métier. Plus particulièrement, un système du type pierre à briquer ou une céramique piézoélectrique du type de celle utilisée dans les allumes gaz est utilisable pour la présente invention.

Ainsi, l'interaction entre les deux éléments qui se déplacent permet de créer une impulsion électrique, celle-ci étant typiquement d'une durée de 1 à 50 millisecondes et pouvant atteindre de 10 000 à 50 000 Volts. Un circuit électronique 25 est prévu pour traiter cette impulsion électrique et l'alimenter à l'afficheur 21 pour provoquer une modification de son affichage.

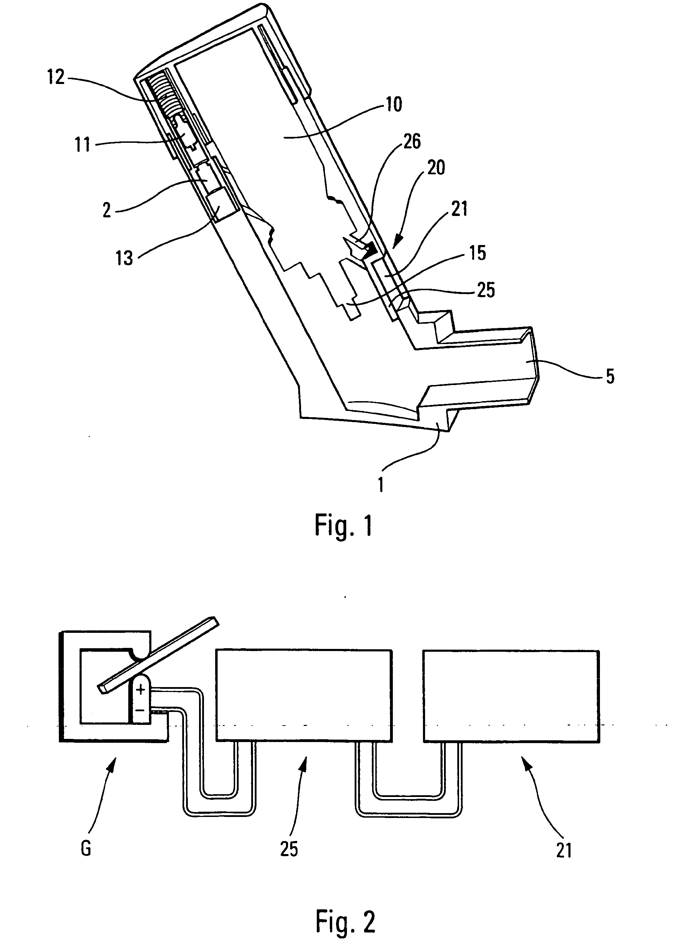

La figure 2 montre de manière schématique le fonctionnement du dispositif d'affichage. Le générateur G (convertisseur électromécanique) créé une impulsion électrique qui est traitée par le circuit électronique 25 avant d'être alimentée à l'afficheur 21. Le générateur fonctionne sans pile ni accumulateur, plus généralement sans aucune énergie externe permanente, l'énergie nécessaire pour créer cette impulsion électrique étant formée par la conversion d'un effort ou déplacement mécanique en un signal électrique.

La figure 1 montre un exemple d'application particulièrement adapté pour le dispositif d'affichage de la présente invention. Dans cet exemple, l'afficheur 21 est utilisé avec un indicateur ou compteur de doses pour un distributeur de produit fluide. Par produit fluide, on entend les produits gazeux, liquides, pâteux ou pulvérulents. Cette mise en oeuvre est particulièrement avantageuse, parce que l'absence de source d'énergie, telle qu'une pile ou un accumulateur, diminue fortement les coûts de fabrication du compteur et rend celui-ci plus fiable. Dans l'exemple représenté, le distributeur comporte un corps 1 dans lequel est monté un réservoir 10 contenant le produit fluide. Un organe de distribution 15, qui dans l'exemple représenté est une valve doseuse, mais qui pourrait tout aussi bien être une pompe, est monté sur le réservoir 10 pour distribuer sélectivement le contenu de celui-ci. Le dispositif représenté sur la figure 1 est un inhalateur buccal comportant un embout buccal 5 à travers lequel le produit est distribué. Bien entendu, tout autre type de distributeur pourrait être associé à la présente invention. Lors de l'actionnement du distributeur, le réservoir 10 est généralement déplacé axialement à l'intérieur du corps 1, ce qui a pour effet d'actionner la valve 15. Ce déplacement peut être utilisé pour créer l'impulsion électrique nécessaire pour commander une modification de l'afficheur 21.

La figure 1 montre un exemple de réalisation du générateur d'impulsion, qui est du type pierre à briquet. Ainsi, un percuteur 11 coopérant avec un ressort 12 est destiné à venir percuter un contacteur 2, par exemple une céramique piézoélectrique 2 solidaire d'une enclume 13 lors de l'actionnement du distributeur. Avantageusement, le contacteur 2 est fixe par rapport au corps, mais il est entendu que tout autre système équivalent ou similaire pourrait être utilisé. Ainsi, on pourrait envisager de convertir un frottement ou un autre type de percussion en signal électrique. Ce signal électrique est ensuite transféré par des fils d'alimentation 26 à un circuit électronique 25 qui coopère avec l'afficheur 21 pour le commander et modifier l'affichage et ainsi compter chaque distribution de dose correspondant à chaque actionnement du distributeur. Comme visible sur la figure 1, les dimensions du compteur de doses sont relativement faibles, ce qui permet d'adapter ce compteur de manière simple dans tout distributeur existant sans modifier sensiblement ses dimensions. L'utilisation d'un afficheur permanent est particulièrement avantageux en ce qu'elle limite fortement la consommation d'énergie, et qu'elle permet de se dispenser d'une pile ou d'un accumulateur ou de tout autre source d'énergie permanente pour alimenter l'afficheur.

Bien qu'ayant été représenté en référence à une variante d'utilisation particulière, il est entendu que le dispositif d'affichage de la présente invention est d'application beaucoup plus générale, et n'est pas limité à la variante de réalisation représentée. Au contraire, un homme du métier peut y apporter toute modification utile sans sortir du cadre de la présente invention telle que défini par les revendications annexées.

## Revendications

1. Dispositif d'affichage électronique (20) comportant un afficheur, deux éléments se déplaçant l'un par rapport à l'autre (10, 11, 2), un convertisseur électromécanique et un circuit électronique (25) (21), **caractérisé en ce que** ledit afficheur (21) est permanent de sorte qu'aucune énergie n'est nécessaire pour maintenir l'affichage inchangé, ledit dispositif d'affichage (20) fonctionnant sans pile ni accumulateur, l'énergie nécessaire pour modifier l'affichage étant créée par interaction entre les deux éléments suite au déplacement mutuel, tel qu'un frottement ou une percussion, créant ainsi au moyen du convertisseur électromécanique une impulsion électrique, ladite impulsion étant traitée par le circuit électronique (25) avant d'être envoyée à l'afficheur (21) pour modifier son affichage.

2. Dispositif d'affichage selon la revendication 1, dans lequel ledit afficheur (21) est du type à cristaux liquides (LCD).

3. Dispositif d'affichage selon la revendication 1 ou 2, dans lequel ledit afficheur (21) comporte des cristaux nématiques bistables.

4. Indicateur ou compteur de doses d'un dispositif de distribution de produit fluide, comportant un dispositif d'affichage selon l'une quelconque des revendications précédentes.

5. Dispositif de distribution de produit fluide, comportant un corps (1), un réservoir de produit fluide (10), un organe de distribution (15), tel qu'une pompe ou une valve, et un compteur de doses pour compter le nombre de doses distribuées ou restant à distribuer du réservoir (10), **caractérisé en ce que** ledit compteur de doses comporte un dispositif d'affichage (20) selon l'une des revendications 1 à 3.

6. Dispositif de distribution selon la revendication 5, dans lequel l'impulsion électrique nécessaire pour modifier l'affichage est créée par un percuteur (11) déplacé contre un contacteur (2) lors de l'actionnement du dispositif de distribution.

7. Dispositif de distribution selon la revendication 6, dans lequel ledit contacteur (2) est fixe par rapport au corps (1) et ledit percuteur (11) coopère avec un ressort (12).

## Claims

1. An electronic display device (20) including a display member (21), two elements (10, 11; 1, 2) moving relative to each other, an electromechanical converter and an electronic circuit (25), said device being **characterized in that** said display member (21) is permanent so that no energy is required to keep the display unchanged, said display device (20) operating without a battery, the energy required to change the display being created by interaction between the two elements further to relative movement, such as a friction or by an impact, thereby creating an electric pulse by means of the electromechanical converter, said pulse being processed by the electronic circuit (25) before being applied to the display member (21) in order to change its display.

2. A display device according to claim 1, in which said display member (21) is of the liquid crystal display (LCD) type.

3. A display device according to claim 1 or claim 2, in which said display member (21) includes bistable nematic crystals.

4. Dose indicator or counter for a fluid dispenser device, comprising a display device according to any preceding claim.

5. A fluid dispenser device comprising: a body (1); a fluid reservoir (10); a dispenser member (15), such as a pump or a valve; and a dose counter for counting the number of doses that have been dispensed or that remain to be dispensed from the reservoir (10), said device being **characterized in that** said dose counter includes a display device (20) according to any one of claims 1 to 3.

6. A dispenser device according to claim 5, in which the electric pulse required to change the display is created by a striker pin (11) that is displaced against a contactor (2) while the dispenser device is being actuated.

7. A dispenser device according to claim 6, in which said contactor (2) is held stationary relative to the body (1), and said striker pin (11) co-operates with a spring (12).

## Patentansprüche

1. Elektronische Anzeigevorrichtung (20), aufweisend eine Anzeige, zwei relativ zueinander verschiebbare Elemente (10,11;1,2), einen elektromechanischen Umformer und eine elektronische Schaltung (25), **dadurch gekennzeichnet, dass** die Anzeige (21) dergestalt dauerhaft ist, dass keinerlei Energie erforderlich ist, um die Anzeige ungeändert aufrecht zu erhalten, wobei die Anzeigevorrichtung (20) ohne Batterie oder Akkumulator arbeitet, wobei die zum Modifizieren der Anzeige erforderliche Energie durch Wechselwirkung zwischen den beiden Elementen durch gegenseitige Verschiebung erzeugt wird, wie etwa durch Reibung oder Stoßeinwirkung, wodurch mittels des elektromagnetischen Umsetzers ein elektrischer Stromstoß erzeugt wird, wobei der Stromstoß durch die elektronische Schaltung (25) aufbereitet wird, bevor er der Anzeige (21) zugeführt wird, um deren Anzeige zu modifizieren.

2. Anzeigevorrichtung nach Anspruch 1, wobei die Anzeige (21) eine Flüssigkristallanzeige (LCD) ist.

3. Anzeigevorrichtung nach Anspruch 1 oder 2, wobei die Anzeige (21) bistabile nematische Kristalle umfasst.

4. Dosisanzeiger bzw. -zähler einer Vorrichtung zur Abgabe eines Fluidprodukts, aufweisend eine Anzeigevorrichtung nach einem der vorangehenden Ansprüche.

5. Vorrichtung zur Abgabe eines Fluidprodukts, aufweisend einen Körper (1), einen Fluidprodukt-Vorratsbehälter (10), ein Abgabeorgan (15), wie etwa eine Pumpe oder ein Ventil, und einen Dosiszähler zum Zählen der Anzahl von Dosen, die vom Vorratsbehälter (10) abgegeben werden oder in diesem verbleiben, **dadurch gekennzeichnet, dass** der Dosiszähler eine Anzeigevorrichtung (20) nach einem der Ansprüche 1 bis 3 umfasst.

6. Abgabevorrichtung nach Anspruch 5, in welcher der elektrische Stromstoß, der zum Modifizieren der Anzeige erforderlich ist, durch einen Schlagbolzen (11) erzeugt wird, der gegen einen Kontaktgeber (2) bei Betätigung der Abgabevorrichtung verschoben wird.

7. Abgabevorrichtung nach Anspruch 6, wobei der Kontaktgeber (2) relativ zum Körper (1) stationär ist, und wobei der Schlagbolzen (11) mit einer Feder (12) zusammenwirkt.
